# EUROPÄISCHE PATENTSCHRIFT

(11) **EP 3 188 769 B1**
(45) Veröffentlichungstag und Bekanntmachung des Hinweises auf die Patenterteilung: **06.01.2021**
(21) Anmeldenummer: 15791487.0
(22) Anmeldetag: 02.09.2015
(51) Int. Cl.: A61M 1/10

(54) **KATHETER**
CATHETER
CATHÉTER

(30) Priorität: 03.09.2014 DE 102014012850
(43) Veröffentlichungstag der Anmeldung: 12.07.2017
(73) Patentinhaber: Novapump GmbH, 07749 Jena (DE)
(72) Erfinder: PFEIFER, Jörg, 07743 Jena (DE); PATZER, Patrick, 07570 Harth-Pöllnitz (DE); REICH, Ronald, 07749 Jena (DE)
(74) Vertreter: Carlsohn, Marc René
(86) Internationale Anmeldenummer: PCT/DE2015/100369
(87) Internationale Veröffentlichungsnummer: WO 2016/034171

(56) Entgegenhaltungen:
- WO-A1-99/58170
- WO-A1-2013/033783
- US-A1- 2007 197 855

## Beschreibung

Die vorliegende Erfindung betrifft einen Katheter zum gerichteten Leiten einer Körperflüssigkeit, insbesondere Blut, der einen Leitungsabschnitt mit einem Innenvolumen, eine erste Öffnung, die das Innenvolumen mit einem Äußeren verbindet, sowie einer distal von der ersten Öffnung angeordneten zweiten Öffnung, die das Innenvolumen mit dem Äußeren verbindet, umfasst und in dessen Betriebszustand die Körperflüssigkeit in dem Innenvolumen zwischen erster und zweiter Öffnung gerichtet geleitet wird.

"Distal" bedeutet im Sinne der Erfindung "zu dem in den Körper eingeschobenen Ende des Katheters hin". Bei dem erfindungsgemäßen Katheter ist also ein von einer ersten Öffnung distal angeordnete zweite Öffnung dem distalen Katheterende (d.h. das Katheterende, das bestimmungsgemäß in den Körper vorgeschoben wird) näher angeordnet als die erste Öffnung. "Proximal" bedeutet im Sinne der Erfindung "vom distalen Katheterende weg". Bei dem erfindungsgemäßen Katheter ist also ein proximales Katheterende dem distalen Katheterende gegenüberliegend angeordnet, und ragt, wenn der Katheter bestimmungsgemäß in den Körper eingesetzt ist, in der Regel aus diesem heraus.

Ein Katheter der eingangs genannten Art ist als Stand der Technik beispielsweise aus WO 2008/113785 A2 bekannt. Er wird vorzugsweise bei eingeschränkter Herzleistung zur Unterstützung des Herzens und des Blutkreislaufes eingesetzt. Insbesondere kann er auch bei einer höhergradigen Aortenklappeninsuffizienz eingesetzt werden. Er dient dazu, die zu leitende Körperflüssigkeit von einem ersten Ort an einen anderen Ort zu transportieren, ohne den Druck der Flüssigkeit am ersten Ort signifikant über den physiologisch vorgegebenen Zustand hinaus zu erhöhen, indem er das Prinzip einer Tauchpumpe realisiert und vorzugsweise durch Einsatz eines Ballonkatheters mit dem Prinzip einer Membranpumpe kombiniert, wobei unter Tauchpumpe eine Pumpe verstanden wird, die in die zu fördernde Flüssigkeit eingetaucht ist und unter Membranpumpe eine Pumpe verstanden wird, deren Antrieb von der zu fördernden Flüssigkeit durch eine Membran getrennt ist. Er ermöglicht so gegenüber der bekannten intraaortalen Ballongegenpulsation einen gerichteten Transport des Körperfluides bei zudem geringerer Belastung des Patienten.

Derartige Katheter können vereinfacht auch als Pumpkatheter bezeichnet werden. Bei einem solchen Pumpkatheter ist es möglich, einen separaten Antrieb zu nutzen. Der Katheter ist dann in seiner Grundform lediglich ein antriebsloser Leitungskatheter. Der Pumpkatheter kann dann beispielsweise bereitgestellt werden, indem nach dem Platzieren des Leitungskatheters ein einstellbarer Verdränger, beispielsweise ein Ballonkatheter einer intraaortalen Ballonpumpe (IABP), in das Innenvolumen des Katheters geschoben wird. Eine Bereitstellung des Katheters ist daher auch ohne Antrieb sinnvoll möglich.

Eine anderer Pumpkatheter ist als Stand der Technik aus der WO 99/58170 A1 bekannt. Dieser ebenfalls nach dem Tauchpumpenprinzip arbeitende Katheter ist ausschließlich zur Anwendung am Linksherzen vorgesehen. In einem Pumpenteil ist ein Flügelrad angeordnet, das für einen kontinuierlichen, d.h. nicht schwallweisen, Blutfluss sorgt und für die empfindlichen Blutbestandteile eine hohe Scherbelastung bedeutet. Der Pumpenteil dieser Pumpe ist innerhalb des Herzens im linken Vorhof angeordnet.

Der Aufwand und die Beeinträchtigung des Patienten beim minimalinvasiven Einführen eines Katheters in den Körper, beispielsweise über Leistengefäße, hängen wesentlich von der Größe, insbesondere dem größten Außendurchmesser des Katheters ab. Aus Sicht des Patienten und des behandelnden Arztes ist es also erwünscht, den Außendurchmesser des Katheters möglichst klein zu wählen. Um andererseits die erforderliche Pumpleistung, d.h. das pro Zeiteinheit zu transportierende Flüssigkeitsvolumen bei gleichzeitig möglichst geringen Belastungen für die transportierte Flüssigkeit zu gewährleisten, ist jedoch ein möglichst großer Innendurchmesser zumindest in dem Abschnitt des Katheters, durch den die Flüssigkeit transportiert werden muss, vorteilhaft.

Der Erfindung liegt daher die Aufgabe zugrunde, einen Katheter der eingangs genannten Art anzugeben, der eine hohe Pumpleistung bei einem geringen Außendurchmesser aufweist.

Diese Aufgabe wird durch einen Katheter gelöst, welcher die in Anspruch 1 angegebenen Merkmale aufweist. In den Unteransprüchen sind vorteilhafte Ausgestaltungen angegeben.

Die Erfindung sieht vor, dass der Katheter einen Leitungsabschnitt mit einem Innenvolumen, eine erste Öffnung, eine zweite Öffnung, eine dritte Öffnung und einen Pumpkammerabschnitt mit einer Pumpkammer umfasst. Innerhalb der Pumpkammer ist Ballon angeordnet und an den Ballon eine Leitung für ein Hilfsfluid zum Aufpumpen des Ballons angeschlossen. Die Leitung für das Hilfsfluid verläuft durch die dritte Öffnung nach außen und ist mit einer Pumpe verbindbar, so dass im Betriebszustand des Katheters die Körperflüssigkeit in dem Innenvolumen zwischen erster und zweiter Öffnung pulsatil gerichtet geleitet wird. Der Leitungsabschnitt umfasst einen Folienschlauch mit einer im Inneren des Folienschlauches verlaufenden Aussteifung, und der Folienschlauch weist einen faltbaren Abschnitt, einen Verbindungsbereich, in dem der Folienschlauch mit der Aussteifung verbunden ist, und einen stabilisierten Abschnitt mit einer Strukturierung auf.

Die Eigenschaft "faltbar" bedeutet im Sinne der Erfindung, dass der bis zu einem vorbestimmten (relativen) Grenz-Unterdruck gegenüber einem Außendruck formstabile Folienschlauch bei höheren negativen Drücken als dem Grenz-Unterdruck instabil wird, wobei die Instabilität bei einer relativen Druckdifferenz ΔP zwischen dem Inneren und dem Äußeren von kleiner als - 500 mmHg, bevorzugt kleiner - 200 mmHg, eintritt.

Dadurch, dass der körperflüssigkeitstransportierende Abschnitt (Leitungsabschnitt) des Katheters einen Folienschlauch umfasst, kann in diesem Abschnitt der Katheter einen größeren Innendurchmesser (vorzugsweise größer als 7 mm und besonders bevorzugt größer als 8 mm) aufweisen als nach dem bisherigen Stand der Technik, womit die pro Zeiteinheit durch das Katheterinnere transportierbare Flüssigkeitsmenge signifikant höher sein kann, als nach dem Stand der Technik. Durch die Bereitstellung des faltbaren Abschnittes ist ein reversibler Faltzustand erreichbar, der ein minimalinvasives Einführen des Katheters in den Körper trotz eines gegenüber dem Stand der Technik vergrößerten Innendurchmessers des distalen Abschnitts bequem ermöglicht. Das ist insbesondere innerhalb der Kardiologie vorteilhaft, da erfahrungsgemäß bei einem verringerten Implantationsdurchmesser des Herzkatheters weniger Patienten eines bestimmten Patientenkollektivs aufgrund ihres individuellen Gefäßinnendurchmessers von einem Eingriff ausgeschlossen werden müssen bzw. ein höherer Patientenanteil einer Akutbehandlung zugeführt werden kann, ohne dass zusätzlich ein Gefäßspezialist unterstützend anwesend sein muss. Zudem kann die Sicherheit der Implantation und Explantation durch den geringeren Implantationsdurchmesser verbessert werden. Bei Anwendungen in der Kardiologie, bei denen der Katheter beispielsweise minimalinvasiv über ein Leistengefäß in das Herz eingeführt wird, kann der Katheter aufgrund des faltbaren Abschnittes deutlich gewebeschonender im Herzen und insbesondere im Bereich einer Herzklappe vorgeschoben werden als es mit konventionellen Herzkathetern bisher möglich gewesen ist.

Der faltbare Abschnitt kann den Leitungsabschnitt umfassen.

Vorzugsweise ist der faltbare Abschnitt im gebrauchsfertigen (d.h. in den Körper einsetzbaren) Zustand des Katheters gefaltet. Damit kann eine weiter verbesserte Einführbarkeit in den Patientenkörper erreicht werden. Die Faltung kann zufällig oder "geordnet" nach einem vorbestimmten Muster bzw. entlang vorbestimmter Faltungslinien sein. Beispielsweise kann es vorgesehen sein, dass das Folienmaterial des faltbaren Abschnittes spiralig oder entlang einer oder mehrerer längs verlaufender Faltungslinien gefaltet ist. Die Faltung kann durch eine über den faltbaren Abschnitt geschobene, entfernbaren Einführhülse aufrechterhalten sein. Auf diese Weise wird es ermöglicht, den Katheter im gefalteten (komprimierten) Zustand des faltbaren Abschnittes über einen Zugang an seinen Bestimmungsort im Körper vorzuschieben und dann dort durch Entfernen der Einführhülse zu entfalten.

Die Verbindung des Folienschlauches mit der Aussteifung und/oder mit einem benachbarten Katheterabschnitt kann beispielsweise durch Verschweißen (beispielsweise Kaltverschweißen oder Ultraschallschweißen) oder durch Verkleben erfolgen.

Der stabilisierte Abschnitt weist einen erhöhten Knickwiderstand auf. Mit anderen Worten weist der stabilisierte Abschnitt eine erhöhte Formstabilität auf. Auf diese Weise kann im in den Körper eingesetzten Zustand des Katheters ein Abknicken des Folienschlauches beispielsweise an einem Ort, an dem der Schlauch aus anatomischen/physiologischen Gründen eine enge Schleife beschreibt, wirkungsvoll verhindert werden. Ein solches Abknicken ist unerwünscht, weil durch den an der Abknickstelle verminderten inneren Schlauchquerschnitt die Menge Körperflüssigkeit, die pro Zeiteinheit durch den Folienschlauch transportierbar ist, erheblich herabgesetzt sein kann. Beispielsweise kann der Folienschlauch, wenn der Katheter zur Behandlung am Rechtsherz eingesetzt ist, innerhalb der rechten Herzkammer angeordnet sein und mit seinem distalen Ende bis in die Lungenschlagader hineinreichen. In diesem Fall beschreibt der Folienschlauch im Bereich der rechten Herzkammer eine enge Schlaufe. Mittels einer entsprechenden abschnittsweisen Stabilisierung des Folienschlauches im Bereich der Schlaufe lässt sich ein Abknicken des Schlauches an dieser Stelle wirkungsvoll verhindern.

Wie weiter unten noch ausführlich beschrieben wird, ist der Katheter für einen pulsatilen Betrieb eingerichtet. Die mit dem pulsatilen Betrieb typischerweise einhergehenden Druckschwankungen führen ebenfalls nicht zu einem (vollständigem) Abknicken des stabilisierten Abschnittes. Damit wird die Betriebssicherheit des Katheters insgesamt wesentlich verbessert.

Der faltbare Abschnitt kann den stabilisierten Abschnitt umfassen.

Die Strukturierung des stabilisierten Abschnittes kann bevorzugt eine rippenförmige Profilierung sein. Damit ist eine effektive Stabilisierung auf einfache Art und Weise möglich. Durch die Ausführung der Rippengröße, der Rippenabstände etc. ist der Knickwiderstand einstellbar. Die Rippen können beispielsweise quer zur Längsrichtung des Folienschlauches oder spiralig angeordnet sein. Bei einer Anordnung quer zur Längsrichtung des Folienschlauches sind die Rippen jeweils geschlossene Ringe. Bei einer spiraligen Anordnung sind eine oder mehrere Rippen in Längsrichtung des Folienschlauches wendelförmig angeordnet. Zusätzlich oder alternativ ist es möglich, dass die Folie des Folienschlauches innerhalb des stabilisierten Abschnitts dicker, beispielsweise um ein Fünftel oder um die Hälfte dicker ausgeführt ist, als in einem benachbarten Abschnitt.
Vorteilhaft kann der Katheter eingerichtet sein, derart, dass die Körperflüssigkeit durch die erste Öffnung in das Innenvolumen (vorstehend und nachfolgend auch Katheterinneres genannt) eingesaugt, in dem Innenvolumen in distale Richtung geleitet und durch die zweite Öffnung wieder aus dem Innenvolumen ausgelassen wird. Durch die Auslegung zum Transport der Körperflüssigkeit in distale Richtung ist der Katheter insbesondere auch für Anwendungen zur Unterstützung der Pumpleistung des Rechtsherzens geeignet. Vorteilhaft sind dabei die Öffnungen derart angeordnet und die Länge des Folienschlauches ist so ausgelegt, dass sich, wenn der Katheter im perkutan in den menschlichen Körper eingesetzten Zustand über eine zentrale Vene in das Rechtsherz eingeführt ist, die erste Öffnung im Bereich der rechten Herzkammer befindet und der Folienschlauch sich von der rechten Herzkammer bis in die Lungenarterie erstreckt, sodass die zweite Öffnung im Bereich der Lungenarterie angeordnet ist. Somit kann zur Unterstützung des rechten Ventrikels Blut im Bereich der rechten Herzkammer in den Katheter aufgenommen, in dem Innenvolumen bis zu dem Bereich der Lungenarterie gerichtet geleitet und dort wieder aus dem Katheter ausgelassen werden. Nach einer anderen vorteilhaften Ausführungsform wird das Rechtsherz durch den Leitungsabschnitt des Katheters überbrückt ("bypassed"). In diesem Fall liegt die erste Öffnung in Fließrichtung des Blutstromes vor dem Rechtsherzen, beispielsweise im Bereich der unteren Hohlvene, von wo Blut aufgenommen und in dem Innenvolumen durch das gesamte Rechtsherz hindurch transportiert und im Bereich der Lungenarterie durch die zweite Öffnung wieder aus dem Katheter ausgelassen wird.

Der Folienschlauch hat eine Länge zwischen 10 cm und 30 cm, bevorzugt zwischen 15 cm und 20 cm, und ist idealerweise etwa 17 cm lang; dies gilt insbesondere für den Fall, dass der Katheter bestimmungsgemäß für den Einsatz am Rechtsherz vorgesehen ist.

Der Folienschlauch besitzt eine Wandstärke von insbesondere weniger als 0,6 mm und bevorzugt von weniger als 0,3 mm.

Das Material des Folienschlauches kann einen Kunststoff, vorzugsweise ein Elastomer wie beispielsweise ein Polyurethan oder einen Thermoplasten wie beispielsweise Polyethylen, umfassen. Das Material sollte für intrakorporale Anwendungen geeignet sein.

Wie vorstehend bereits im Einzelnen erläutert, kann der erfindungsgemäße Katheter somit einen Folienschlauch aufweisen, der in Unterabschnitte gegliedert ist, wobei sich die Unterabschnitte beispielsweise jeweils in der Wandstärke (innerhalb des oben angegebenen Bereichs), der Materialzusammensetzung, der Materialdichte, des Knickwiderstandes, des Druckwiderstandes, des Durchmessers und/oder der Strukturierung der inneren und/oder äußeren Oberfläche voneinander unterscheiden können.

Vorzugsweise ist der Folienschlauch zumindest abschnittsweise, insbesondere abseits seiner Enden, exakt oder im wesentlichen radiärsymmetrisch oder rotationssymmetrisch (unendlich-zählig radiärsymmetrisch) um eine Längsachse, insbesondere zylinderförmig, und die erste Öffnung und oder die zweite Öffnung in einer (die Längsachse umgebenden) Mantelfläche, insbesondere einer Zylindermantelfläche, des Folienschlauches angeordnet. Der kleinste Außendurchmesser entspricht dann dem Querschnitt zur Längsachse. Je höherzähliger die Radiärsymmetrie ist, umso geringer kann vorteilhafterweise der kleinste Außendurchmesser sein.

Vorteilhaft ist die Aussteifung durch einem Führungsschlauch, beispielsweise einem marktüblichen Angiographiekatheter oder ähnlichem, gebildet, der ein weiteres Lumen (nachfolgend auch Schlauchinneres genannt) aufweist. Bevorzugt weist der Führungsschlauch einen Außendurchmesser zwischen 0,5 mm und 2 mm auf.

Bevorzugt ist der Führungsschlauch eingerichtet, um zur bestimmungsgemäßen Positionierung des Katheters über einen Führungsdraht geschoben zu werden. Dadurch, dass die Aussteifung zusätzlich die Funktion eines über einen Führungsdraht schiebbaren Führungsschlauches übernimmt, kann der Katheter beispielsweise nach der in der Kardiologie bekannten Seldingertechnik implantiert werden. Der Katheter weist in diesem Fall bevorzugt eine dritte proximale Öffnung auf und der Führungsschlauch verläuft von dieser proximalen Öffnung durch den Katheter bis zu der zweiten Öffnung. Das distale Ende des Führungsschlauchs kann durch die zweite Öffnung hindurchtreten. Die Spitze des Führungsschlauchs ist bevorzugt zurückgebogen.

Vorteilhaft umfasst das distale Ende des Führungsschlauches eine Medikamentenöffnung. Alternativ oder zusätzlich kann auch im Bereich der zweiten Öffnung eine (zusätzliche) Medikamentenöffnung angeordnet sein. Die Medikamentenöffnung verbindet das Schlauchinnere (des Führungsschlauches) mit dem Äußeren, so dass über diese Medikamentenöffnung das Schlauchinnere mit dem Äußeren kommuniziert. Auf diese Weise wird es ermöglicht, im in einen Körper eingesetzten Zustand des Katheters dem Körper über den Führungsschlauch ein Medikament zu applizieren, das durch die Medikamentenöffnung aus dem Katheter austritt und beispielsweise lokal in dem Bereich des Körpers, der die Medikamentenöffnung umgibt, seine Wirkung schneller und/oder gezielter entfalten kann.

Vorteilhaft kann der Folienschlauch eine Mehrzahl von zweiten Öffnungen aufweisen. Die zweiten Öffnungen können zumindest teilweise von dem distalen Ende des Folienschlauches beabstandet angeordnet sein. Durch die Anordnung mehrerer zweiter Öffnungen kann deren (Gesamt-)Öffnungsquerschnitt wirkungsvoll erhöht werden, wodurch die nach distal transportierte Körperflüssigkeit mit geringeren lokalen Drücken wieder aus dem Katheterinneren ausgelassen werden kann. Vorteilhaft können somit die auf den Folienschlauch, die Körperflüssigkeit und das die zweiten Öffnungen umgebende Körpergewebe einwirkenden Kräfte verringert werden.

Bevorzugt weist der Folienschlauch einen distalen, insbesondere blasenförmig erweiterten Abschnitt mit einem (gegenüber dem benachbarten Abschnitt) vergrößerten mittleren Außendurchmesser auf und die zweiten Öffnungen sind verteilt innerhalb dieses Abschnitts angeordnet. Durch eine derartige Anordnung der zweiten Öffnungen wird bewirkt, dass die Körperflüssigkeit in unterschiedliche Richtungen aus dem Katheter austritt, womit die bei dem nicht kontinuierlichen, pulsatilen, d.h. stoß- bzw. schwallweisen, Transport der Körperflüssigkeit die auf den Folienschlauch, die Körperflüssigkeit und das die zweiten Öffnungen umgebende Körpergewebe einwirkenden Kräfte noch weiter verringert werden, wobei insbesondere ein "Schlagen" des distalen Endes des Folienschlauches aufgrund der mit dem pulsatilen Transport verbundenen Druckschwankungen (Systole und Diastole bei der Verwendung des Katheters als Herzkatheter) weitgehend vermieden wird.

Der Katheter umfasst, wie bereits erwähnt, (neben dem Leitungsabschnitt) einen Pumpkammerabschnitt. Zwischen dem Leitungsabschnitt und dem Pumpkammerabschnitt können ein oder mehrere Verbindungsabschnitte angeordnet sein. Die Hülle des Pumpkammerabschnittes kann durch den Folienschlauch gebildet sein. Der Pumpkammerabschnitt kann ganz oder teilweise ein Unterabschnitt des Leitungsabschnittes sein. Der Pumpkammerabschnitt kann ganz oder teilweise ein Unterabschnitt des faltbaren Abschnittes sein. Zweckmäßigerweise ist die erste Öffnung im Bereich eines Endes des Leitungsabschnitts angeordnet und die zweite Öffnung im Bereich eines entgegengesetzten Endes des Leitungsabschnitts angeordnet.

In einer bevorzugten Ausführungsform ist die erste Öffnung im Bereich des Pumpkammerabschnittes angeordnet. In diesem Fall bildet der Pumpkammerabschnitt einen Teil des Leitungsabschnitts, so dass die Pumpkammer Teil der Leitung ist.

In einer weiteren geeigneten Ausführungsform kann die erste Öffnung jedoch auch abseits des Pumpkammerabschnitts in dem Leitungsabschnitt angeordnet sein.

Typischerweise weist der Katheter im Bereich des Pumpkammerabschnitts einen größeren Innendurchmesser auf als im an den Pumpkammerabschnitt angrenzenden Bereich des Leitungsabschnitts. Insbesondere weist der Pumpkammerabschnitt einen mittleren Innendurchmesser von größer als 15 mm auf.

Wie erwähnt, umfasst der Pumpkammerabschnitt eine Pumpkammer. Die Pumpkammer weist bevorzugt einen (aufstellbaren) Rahmen auf. Das Material des Rahmens umfasst vorzugsweise eine Zusammensetzung, die eine Formgedächtnislegierung, insbesondere Nitinol, ein Formgedächtnispolymer oder eine Formgedächtniskeramik aufweist. Der Rahmen ist im Wesentlichen schlauchförmig ausgebildet. Vorzugsweise ist der Rahmen zumindest abschnittsweise, insbesondere abseits seiner Enden, oder zumindest seiner äußeren Einhüllenden exakt oder im Wesentlichen radiärsymmetrisch oder rotationssymmetrisch (unendlich-zählig radiärsymmetrisch) um eine Längsachse, insbesondere zylinderförmig angeordnet. Insbesondere kann der Rahmen ein aufstellbarer Stent sein. Mit anderen Worten kann der Pumpkammerabschnitt oder zumindest die Pumpkammer faltbar sein. Der faltbare Abschnitt kann somit den Pumpkammerabschnitt bzw. die Pumpkammer umfassen. Der Rahmen ist bevorzugt im Innenraum der Pumpkammerangeordnet.

Die Pumpkammer ist vorzugsweise zwischen 150 mm und 300 mm lang.

Außerdem weist der Katheter wie schon erwähnt, proximal eine dritte Öffnung auf, so dass ein Antrieb, insbesondere ein Ballon eines Ballonkatheters, insbesondere eines intraaortalen Ballonpumpkatheters (IABP), durch die dritte Öffnung in den Innenraum des Katheters bis zu einer vorbestimmten Zielposition relativ zu dem Katheter schiebbar ist. Bevorzugt entspricht die vorbestimmte Zielposition der Pumpkammer, d.h. bestimmungsgemäß wird der Ballon bevorzugt in der Pumpkammer angeordnet. Zweckmäßigerweise ist der Antrieb so durch die dritte Öffnung schiebbar, dass diese fluiddicht (d.h. insbesondere zumindest gegenüber einem maximalen Blutdruck) verschlossen ist. Als Antrieb in Form eines Verdrängers kann beispielsweise ein Katheter gemäß US 5,460,607 A eingesetzt werden. Der im Innenraum angeordnete Antrieb ist über eine durch die dritte Öffnung hindurchführende Leitung mit einer externen Energiequelle verbunden; im Falle eines Ballonkatheters beispielsweise über eine Hilfsfluidleitung mit einer Pumpkonsole (Pumpe), die den Ballon mit einem Hilfsfluid vorzugsweise stoßartig befüllen und entleeren kann. Aufgrund der verdrängenden Wirkung des befüllten Ballons wird ein gerichteter Transport der Körperflüssigkeit ermöglicht. Der Antrieb kann beispielsweise hinsichtlich der Frequenz der Befüllvorgänge des Ballons mit Hilfsfluid und/oder des Volumens an Hilfsfluid pro Befüllvorgang einstellbar sein.

In einer besonders vorteilhaften Ausgestaltungsvariante ist der Katheter so konfektioniert, dass der Katheter eine Pumpkammer aufweist, in welcher der Ballon eines IAB-Katheters fest angeordnet ist. Mittels einer Leitung für ein Hilfsfluid, die durch die dritte Öffnung des Katheters nach außen verläuft, ist der Ballon mit einer externen Pumpe, insbesondere einer sogenannten IABP-Pumpkonsole, verbindbar. Dadurch ist der Katheter ohne die zusätzlichen Arbeitsschritte des nachträglichen Einbringens eines separaten Verdrängers in das zu leitende Fluid und des Einschiebens des Ballons in den Innenraum des Leitungskatheters einsatzbereit. Die Implantationsdauer wird dadurch verringert.

Als Hilfsfluid zum Befüllen des Ballons ist vorzugsweise Helium vorgesehen.

An der ersten Öffnung und/ oder der zweiten Öffnung kann ein Rückschlagventil (zum Ermöglichen einer ausschließlich unidirektionalen Strömung zwischen dem Innenvolumen und dem den Katheter umgebenden Außenraum) angeordnet sein. Das Rückschlagventil ist vorzugsweise als Folienventil gemäß der DE 10 2014 003 153.5 ausgebildet. Bevorzugt sind innerhalb des Pumpkammerabschnittes eine Vielzahl von Folienventilen (insbesondere mehr als 50 oder sogar mehr als 100 Folienventile) angeordnet. In diesem Fall sind die einzelnen Folienventile bevorzugt in sich entlang des Pumpkammeraschnittes erstreckenden, radial gleichverteilten Reihen angeordnet.

Zusätzlich oder alternativ ist es möglich, dass der faltbare Abschnitt bzw. ein Unterabschnitt des faltbaren Abschnitts eine Ventilfunktion bereitstellt. Diese ist vorzugsweise in Kombination mit einem pulsatilen Betrieb des Katheters wirksam, wobei die durch den stoßweisen Transport der Körperflüssigkeit verbundenen periodischen Änderungen der Druckverhältnisse im Katheterinneren zum periodischen Kollabieren und anschließenden Aufrichten des faltbaren Abschnittes / Unterabschnittes führen. Beispielsweise kann es vorgesehen sein, in dem Katheterinneren befindliche Körperflüssigkeit mittels eines befüllbaren Ballons, der proximal von dem faltbaren Abschnitt angeordnet ist, pulsatil in distale Richtung zu pumpen, indem die Körperflüssigkeit durch die Volumenvergrößerung des Ballons beim Befüllen nach distal verdrängt wird und durch die distal von dem faltbaren Abschnitt angeordnete zweite Öffnung aus dem Katheterinneren entweicht. Der faltbare Abschnitt / Unterabschnitt befindet sich dabei aufgrund des momentan in dem Katheterinneren herrschenden Überdrucks im aufgerichteten Zustand. Anschließend wird der Ballon evakuiert, wodurch sich im Katheterinneren ein Unterdruck einstellt, der zum Kollabieren des faltbaren Abschnittes / Unterabschnittes führt. Durch den dadurch stark verminderten inneren Schlauchquerschnitt im Bereich des faltbaren Abschnittes / Unterabschnittes wird die Ventilwirkung bereitgestellt, die ein Rückfluss von nach distal verdrängter Körperflüssigkeit in proximale Richtung wirkungsvoll verhindert.

Nachfolgend wird die Erfindung anhand von Zeichnungen noch näher erläutert. Darin zeigt
Fig. 1 einen Leitungsabschnitt des erfindungsgemäßen Katheters mit einem Folienschlauch, der einen faltbaren Abschnitt aufweist, der einen stabilisierten Abschnitt umfasst,
Fig. 2 einen Teil des stabilisierten Abschnittes des Folienschlauchs gemäß Fig. 1,
Fig. 3 eine Ausführungsform des Katheters mit einem blasenförmig erweiterten distalen Abschnitt mit einer Mehrzahl von zweiten Öffnungen,
Fig. 4 einen Teil des Folienschlauches eines erfindungsgemäßen Katheters mit einer distalen blasenförmig erweiterten Abschnitt,
Fig. 5 beispielhaft die Lage eines erfindungsgemäßen Katheters im rechten Herzen eines menschlichen Patienten (Zugang über die obere Hohlvene), sowie
Fig. 6 ein weiteres Lagebeispiel (Zugang über die untere Hohlvene) eines erfindungsgemäßen Katheters im rechten Herzen eines menschlichen Patienten.

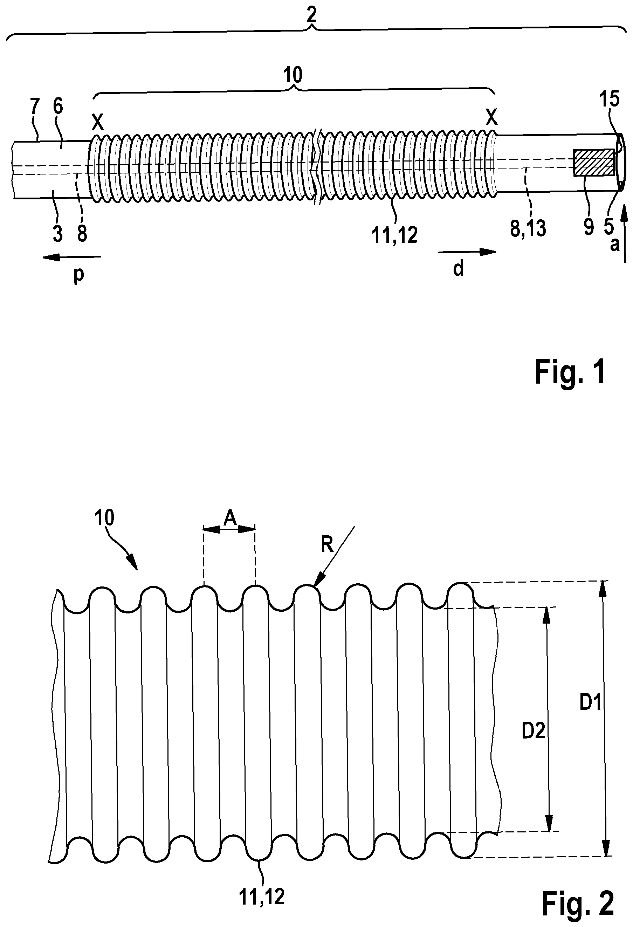

Fig. 1 zeigt den Leitungsabschnitt (2) eines erfindungsgemäßen Katheters (1). Die Richtungspfeile (p) und (d) veranschaulichen die Orientierungen distal d und proximal p. Der Leitungsabschnitt (2) umfasst einen Folienschlauch (6), der ein Innenvolumen (3) umhüllt. Das Innenvolumen (3) kommuniziert mit dem Äußeren X über eine erste Öffnung (4; nicht dargestellt) und eine zweite Öffnung (5). Die erste Öffnung (4) ist am proximalen Ende des Leitungsabschnittes (2) angeordnet und die zweite Öffnung ist am distalen Ende des Leitungsabschnittes (2) angeordnet. Im Inneren (3) des Folienschlauches verläuft eine Aussteifung (8). Zur besseren Übersichtlichkeit ist die Aussteifung (8) gestrichelt gezeichnet. Die Aussteifung (8) ist nahe des distalen Endes des Katheters (1) in einem Verbindungsbereich (9) mit dem Folienschlauch (6) verbunden. In dem Ausführungsbeispiel der Fig. 1 ist die Aussteifung (8) als Führungsschlauch (13) ausgebildet. Der Führungsschlauch (13) ist eingerichtet, um über einen Führungsdraht geschoben zu werden und weist dazu an seinem distalen Ende eine Schlauchöffnung (15) auf. Damit kann der Katheter auf einfache Weise gemäß der Seldingertechnik in den Körper eines Patienten implantiert werden. Der Folienschlauch weist einen faltbaren Abschnitt (7) auf. In dem Ausführungsbeispiel der Fig. 1 umfasst der faltbare Abschnitt (7) zusätzlich einen stabilisierten Abschnitt (10). Der faltbare Abschnitt ist dadurch gekennzeichnet, dass er zur besseren Einführbarkeit des Katheters in den Patientenkörper gefaltet in eine Einführhülse (nicht dargestellt) verpackt werden kann. Die Einführhülse weist einen physiologisch günstigen Außendurchmesser von beispielsweise weniger als 20 French auf. Nach Punktierung und Dilatation eines Leistengefäßes wird der in die Einführhülse verpackte Katheter in das Gefäß vorgeschoben. Anschließend wird die Einführhülse rückwärts wieder aus dem Gefäß entfernt, wodurch der faltbare Abschnitt (7) entpackt wird. Aufgrund seiner relativen Flexibilität kann der faltbare Abschnitt (7) anschließend gewebeschonend an seinen Bestimmungsort, beispielsweise den rechten Ventrikel (24), weiter vorgeschoben werden.

Der stabilisierte Abschnitt (10) ist rippenförmig strukturiert. Dies ist besonders gut bei Fig. 2 zu erkennen, die einen Ausschnitt des Folienschlauches der Fig. 1 zeigt. Die Rippen sind periodisch quer zur Längsrichtung in Form von geschlossenen Ringen, also nicht helikal, angeordnet. Der Nenndurchmesser des stabilisierten Abschnittes entspricht dem Durchmesser an dem Kamm einer Rippe (D1), der Kerndurchmesser des stabilisierten Abschnittes entspricht dem Durchmesser Grund einer Rippe (D2). Im Ausführungsbeispiel der Fig. 2 beträgt der Nenndurchmesser (D1) 9,6 mm und der Kerndurchmesser (D2) 8,1 mm. Der Abstand zwischen zwei Rippen (Rippenperiode A) beträgt in dem vorliegenden Ausführungsbeispiel 1,6 mm. Der Radius (R) einer Rippe beträgt 0,45 mm.

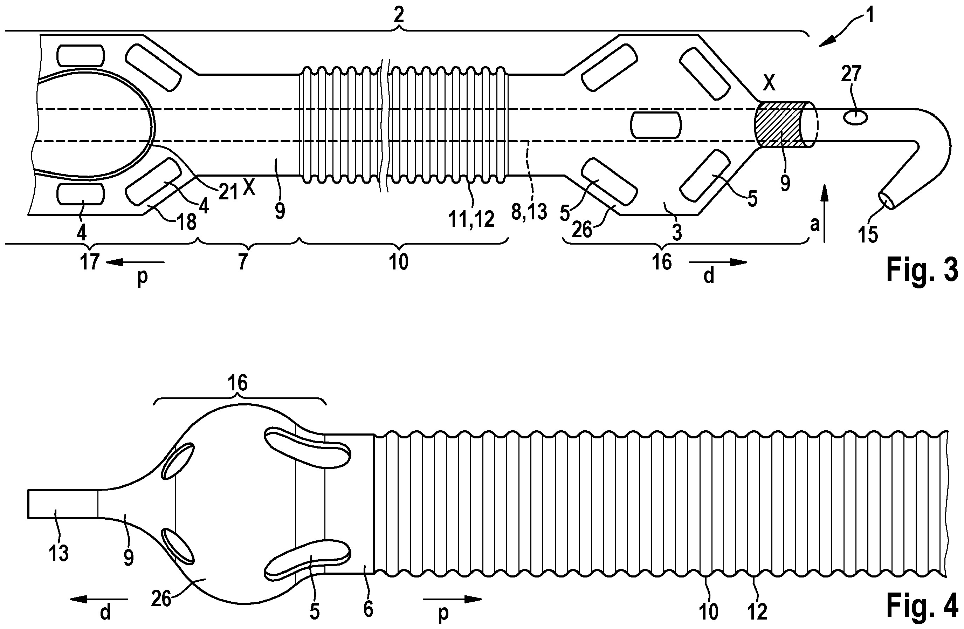

Fig. 3 zeigt eine Ausführungsform des Katheters (1). In der Darstellung der Fig. 3 ist unter anderem der proximal von dem Folienschlauch (6) angeordnete Pumpkammerabschnitt (17) dargestellt. Der Pumpkammerabschnitt (17) umfasst eine Pumpkammer (18) und einen innerhalb der Pumpkammer (18) angeordneten Ballon (21) eines Ballonkatheters. Der Ballon (21) ist an eine Leitung für ein Hilfsfluid angeschlossen, die durch eine dritte proximale Öffnung des Katheters (aus Übersichtlichkeitsgründen nicht gezeigt) nach außen verläuft. Über diese Leitung ist der Ballon (21) mit einer externen Pumpe, insbesondere einer sogenannten IABP-Pumpkonsole, verbindbar. Der Ballon (21) wird pulsatil betrieben, d.h. stoßartig mit dem Hilfsfluid befüllt und entleert und dient somit als Antrieb für den gerichteten Transport der Körperflüssigkeit. Der Katheter (1) gemäß der Fig. 3 kann somit vorteilhaft zur Durchführung des intraaortalen Ballongegenpulsationsverfahrens verwendet werden. Weiterhin weist der Katheter (1) einen blasenförmig erweiterten distalen Abschnitt (16; 26) auf, der eine Mehrzahl von zweiten Öffnungen (5) beinhaltet. Diese sind so verteilt innerhalb des distalen Abschnitts (16; 26) angeordnet, dass die durch den Leitungsabschnitt (29 transportierte Körperflüssigkeit in unterschiedliche Richtungen aus den zweiten Öffnungen (5) ausströmt. Dadurch wird insbesondere bei einem pulsatilen Transport der Körperflüssigkeit die auf den Folienschlauch (6), die Körperflüssigkeit und das die zweiten Öffnungen (5) äußerlich umgebende Körpergewebe einwirkende Kraft verringert, wobei insbesondere ein "Schlagen" des Folienschlauches (6) aufgrund der mit dem pulsatilen Transport verbundenen Druckschwankungen (Systole und Diastole bei der Verwendung des Katheters (1) als Herzkatheter) weitgehend vermieden wird.

Wie in der Fig. 4 gezeigt ist, kann der blasenförmig erweiterte distale Abschnitt (16; 26) besonders bevorzugt direkt proximal an den Verbindungsbereich (9) angrenzen. Der Übergang von dem Verbindungsbereich (9) zu dem distalen Abschnitt (16; 26) kann äußerlich so ausgeführt sein, dass ein glatter Übergang geschaffen ist, der ein leichtes Vorschieben des Katheters (1) ermöglicht. Im Katheterinneren bildet der distale Abschnitt ein im Wesentlichen kugeliges Endstück.

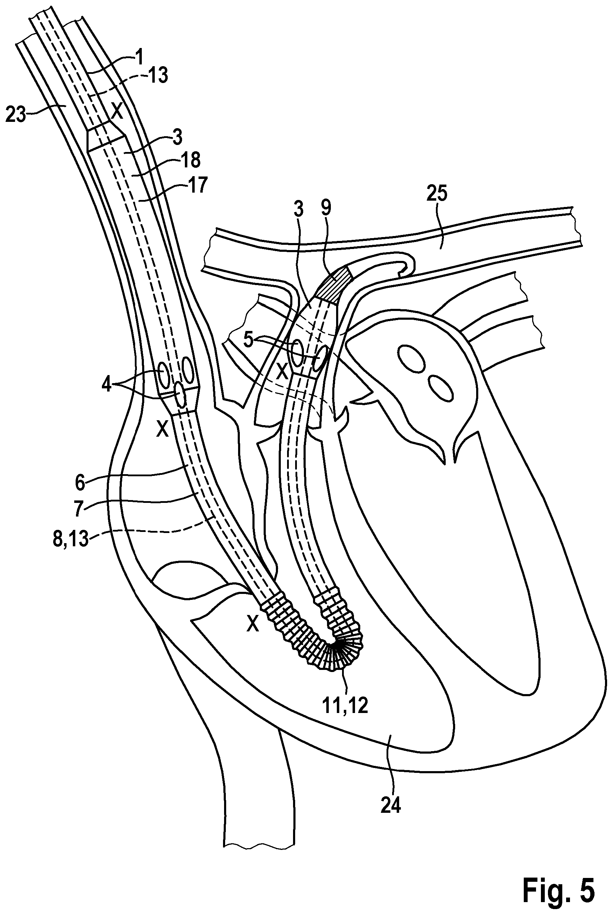

Die Fig. 5 zeigt eine typische Anwendung des Katheters (1) als Blutpumpe. Zur kardiologischen Akutbehandlung ist der Katheter minimalinvasiv über einen venösen Zugang in der Halsgegend in einen Patienten implantiert. Der Zugang über die obere Hohlvene, wie in Fig. 5 gezeigt, ist rein beispielhaft zu verstehen und vorliegend nur aus Gründen der besseren Darstellbarkeit gewählt. In der Praxis erfolgen Implantationen von Herzkathetern dagegen häufig über einen Zugang in der Leistengegend. Der distale Leitungsabschnitt (2) des Katheters ist in die rechte Herzkammer (24) vorgeschoben. In der oberen Hohlvene (23) befindet sich der Pumpkammerabschnitt (17) mit der Pumpkammer (18). Die Pumpkammer (18) ist Teil des Leitungsabschnittes (2). Die Pumpkammer ist für einen pulsatilen Betrieb eingerichtet, d.h. innerhalb der Pumpkammer ist ein Ballon (21) eines Ballonkatheters angeordnet (nicht gezeigt). Der Ballon (21) wird gemäß dem Ausführungsbeispiel der Fig. 4 pulsatil betrieben, d.h. stoßartig mit dem Hilfsfluid befüllt und entleert und dient somit als Antrieb für einen gerichteten Transport des Blutes. Innerhalb des Pumpkammerabschnittes (17) sind erste Öffnungen (4) angeordnet. Durch die ersten Öffnungen (4) wird das Blut in den Katheter (1) eingesaugt und im Katheterinneren (3) des Leitungsabschnittes (2) pulsatil entsprechend der Antriebsfrequenz des Ballons (die beispielsweise einem EKG-Signal folgen kann) gerichtet nach distal zu den zweiten Öffnungen (5) transportiert, wo es den Katheter wieder verlässt. Das distale Ende des Katheters (1) reicht bis in die Lungenarterie (25). Der Leitungsabschnitt (2) des Katheters (1) durchspannt (überbrückt) somit das gesamte Rechtsherz. Die zweiten Öffnungen (5) liegen in dem Ausführungsbeispiel der Fig. 5 im Pulmonalstamm. Der Leitungsabschnitt (2), d.h. sowohl der Pumpkammerabschnitt (17) als auch der sich distal anschließende Pumpenschlauch (6), ist faltbar ausgeführt und bildet somit einen faltbaren Abschnitt (7). Innerhalb der Pumpkammer (18) ist ein aufstellbarer Rahmen (19) angeordnet, der für eine für den pulsatilen Betrieb ausreichende Steifigkeit der Pumpkammer (18) sorgt. Zum Einsetzen des Katheters (1) in den Körper ist der Leitungsabschnitt (2) gefaltet in eine Einführhülse verpackt (nicht gezeigt). Der so verpackte Katheter wird über einen Zugang in der oberen Hohlvene vorgeschoben, bis die Lage des Leitungsabschnittes (2) der in Fig. 5 gezeigten Lage entspricht und der Leitungsabschnitt (2) das Herz durchdringt. Der Einführhülse wird anschließend zurückgezogen, wodurch sich der Rahmen (19) aufstellt und der Leitungsabschnitt (2) sich insgesamt entfaltet. Aufgrund der Ausbildung des Leitungsabschnittes (2) als faltbarer Folienschlauch (6) werden die empfindlichen Herzklappen bei der Implantation und Explantation kaum geschädigt. Aufgrund des stabilisierten Abschnittes (10) wird ein Abknicken des Folienschlauches (6) in anatomisch kritischen Bereichen innerhalb des Herzens verhindert.

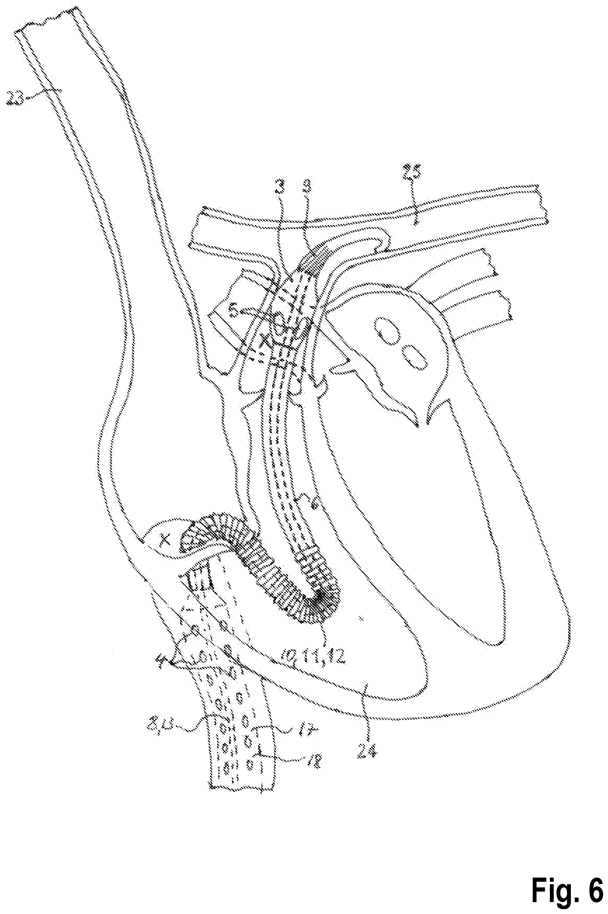

In Fig. 6 ist der Katheter (1), der strukturell dem Katheter gemäß der Fig. 5 entspricht, jedoch in seinen Unterabschnitten abweichende Abmessungen aufweisen kann, über einen alternativen Zugang eines Leistengefäßes verlegt und so weit vorgeschoben, dass sich der Pumpkammerabschnitt (17) mit den ersten Öffnungen (4) in seiner funktionalen Position im Bereich der unteren Hohlvene befindet. Der distal von dem Pumpkammerabschnitt angeordnete Pumpenschlauch (6) durchspannt das rechte Atrium und den rechten Ventrikel und reicht mit seinem distalen Ende wiederum bis in die Pulmonalarterie. Die zweiten Öffnungen (5) sind im Bereich des Pulmonalstamms angeordnet. Wie schon erwähnt, stellt diese Verlegevariante in der Praxis den Standardfall dar. Der Katheter, insbesondere die Länge des Leitungsabschnittes (2), des Pumpkammerabschnittes (17), des distalen Pumpenschlauches und/oder die Lage des stabilisierten Abschnittes (10) kann für eine optimale Passform speziell an diese Verlegevariante angepasst sein. Beispielsweise kann der Leitungsabschnitt 2 (inkl. des Pumpkammerabschnittes 17) eine Länge von 450 mm aufweisen; wobei der Pumpkammerabschnitt (17) etwa 250 mm lang ist und der sich distal anschließende Pumpenschlauch / Folienschlauch (6) etwa 200 mm lang ist. Die ersten Öffnungen (4) sind als Folienventile ausgebildet, die in 5 radial verteilten Reihen zu je 20 Ventilen entlang des Pumpkammerabschnittes (17) angeordnet sind.

### Bezugszeichenliste

- 1: Katheter
- 2: Leitungsabschnitt
- 3: Innenvolumen
- 4: Erste Öffnung
- 5: Zweite Öffnung
- 6: Folienschlauch
- 7: Faltbarer Abschnitt
- 8: Aussteifung
- 9: Verbindungsbereich
- 10: Stabilisierter Abschnitt
- 11: Strukturierung
- 12: rippenförmige Strukturierung
- 13: Führungsschlauch
- 14: Führungsdraht
- 15: Schlauchöffnung
- 16: distaler Abschnitt
- 17: Pumpkammerabschnitt
- 18: Pumpkammer
- 19: Rahmen
- 20: Dritte Öffnung
- 21: Ballon
- 22: Hilfsfluid-Leitung
- 23: obere Hohlvene
- 24: rechte Herzkammer
- 25: Lungenarterie
- 26: blasenförmig erweiterter Abschnitt
- 27: Medikamentenöffnung
- d: distal
- p: proximal
- A: Rippenperiode (Abstand Rippe-Rippe)
- D1: Nenndurchmeser (Rippenspitze)
- D2: Kerndurchmesser (Rippengrund)
- R: Radius Rippe
- X: Äußeres

## Patentansprüche

1. Katheter (1) zum gerichteten Leiten einer Körperflüssigkeit, insbesondere Blut, umfassend:
- einen Leitungsabschnitt (2) mit einem Innenvolumen (3),
- einen Pumpkammerabschnitt (17),
- eine erste Öffnung (4), die das Innenvolumen (3) mit einem Äußeren (X) verbindet,
- eine distal von der ersten Öffnung (4) angeordnete zweite Öffnung (5), die das Innenvolumen (3) mit dem Äußeren (X) verbindet,
- eine dritte Öffnung (20);
wobei der Pumpkammerabschnitt (17) eine Pumpkammer (18) umfasst, in der Pumpkammer (18) ein Ballon (21) angeordnet und an den Ballon (21) eine Leitung für ein Hilfsfluid (22) zum Aufpumpen des Ballons (21) angeschlossen ist und die Leitung für das Hilfsfluid (22) durch die dritte Öffnung (20) nach außen verläuft und mit einer Pumpe verbindbar ist, so dass im Betriebszustand des Katheters (1) die Körperflüssigkeit in dem Innenvolumen (3) zwischen erster (4) und zweiter Öffnung (5) pulsatil gerichtet geleitet wird,
der Leitungsabschnitt (2) einen Folienschlauch (6) mit einer im Inneren des Folienschlauches (6) verlaufenden Aussteifung (8) umfasst,
und wobei der Folienschlauch (6) einen faltbaren Abschnitt (7), einen Verbindungsbereich (9), in dem der Folienschlauch (6) mit der Aussteifung (8) verbunden ist, und einen stabilisierten Abschnitt (10) mit einer Strukturierung (11) aufweist.

2. Katheter (1) nach Anspruch 1, wobei der faltbare Abschnitt (7) im gebrauchsfertigen Zustand des Katheters (1) gefaltet ist.

3. Katheter (1) nach Anspruch 1 oder 2, wobei der stabilisierte Abschnitt (7) eine rippenförmige Profilierung (12) umfasst.

4. Katheter (1) nach einem der obigen Ansprüche, wobei der Katheter (1) eingerichtet ist, derart, dass die Körperflüssigkeit durch die erste Öffnung (4) in das Innenvolumen eingesaugt, in dem Innenvolumen (3) in distale Richtung (d) geleitet und durch die zweite Öffnung (5) wieder aus dem Innenvolumen (3) ausgelassen wird.

5. Katheter (1) nach einem der obigen Ansprüche, wobei der Folienschlauch (6) eine Wandstärke von weniger als 0,6 mm, bevorzugt weniger als 0,3 mm aufweist.

6. Katheter (1) nach einem der obigen Ansprüche, wobei das Material des Folienschlauches (6) einen Kunststoff, bevorzugt ein Polyurethan, umfasst.

7. Katheter (1) nach einem der obigen Ansprüche, wobei die Aussteifung (8) aus einem Führungsschlauch (13) mit einem Schlauchinneren gebildet ist, wobei der Führungsschlauch (13) einen Außendurchmesser zwischen 0,5 mm und 2 mm aufweist.

8. Katheter (1) nach Anspruch 7, wobei der Führungsschlauch (13) eingerichtet ist, um zur bestimmungsgemäßen Positionierung des Katheters (1) über einen Führungsdraht (14) geschoben zu werden.

9. Katheter (1) nach Anspruch 7 oder 8, wobei distale Ende des Führungsschlauches (13) eine Medikamentenöffnung (27) umfasst.

10. Katheter (1) nach einem der vorherigen Ansprüche, wobei der Folienschlauch (6) eine Mehrzahl von zweiten Öffnungen (5) aufweist, die zumindest teilweise von dem distalen Ende des Folienschlauches (6) beabstandet angeordnet sind.

11. Katheter (1) nach Anspruch 10, wobei der Folienschlauch (6) einen distalen, insbesondere blasenförmig erweiterten, Abschnitt (16) aufweist, wobei die zweiten Öffnungen (5) verteilt innerhalb dieses Abschnitts angeordnet sind.

12. Katheter (1) nach einem der vorhergehenden Ansprüche, wobei die Pumpkammer (18) einen Rahmen (19) aufweist, mit einer Zusammensetzung des Rahmens, die eine Formgedächtnislegierung, insbesondere Nitinol, ein Formgedächtnispolymer oder eine Formgedächtniskeramik umfasst.

## Claims

1. Catheter (1) for directionally conducting a body fluid, in particular blood, comprising:
- a line portion (2) having an inner volume (3);
- a pump chamber portion (17);
- a first opening (4) which connects the inner volume (3) to an exterior (X);
- a second opening (5) which is arranged distally from the first opening (4) and connects the inner volume (3) to the exterior (X);
- a third opening (20),
wherein the pump chamber portion (17) comprises a pump chamber (18), a balloon (21) is arranged in the pump chamber (18) and a line for an auxiliary fluid (22) for inflating the balloon (21) is connected to the balloon (21), and the line for the auxiliary fluid (22) extends through the third opening (20) outward and can be connected to a pump such that, when the catheter (1) is in operation, the body fluid is directionally conducted in a pulsatile manner in the inner volume (3) between the first (4) and the second opening (5), the line portion (2) comprises a film tube (6) having a reinforcement (8) extending within the film tube (6),
and wherein the film tube (6) has a foldable portion (7), a connecting region (9) in which the film tube (6) is connected to the reinforcement (8), and a stabilized portion (10) having a structure (11).

2. Catheter (1) according to claim 1, wherein the foldable portion (7) is folded in the ready-to-use state of the catheter (1).

3. Catheter (1) according to either claim 1 or claim 2, wherein the stabilized portion (7) comprises a rib-shaped profile (12).

4. Catheter (1) according to any of the preceding claims, wherein the catheter (1) is configured such that the body fluid is drawn through the first opening (4) into the inner volume, conducted in the inner volume (3) in the distal direction (d) and is discharged again from the inner volume (3) through the second opening (5).

5. Catheter (1) according to any of the preceding claims, wherein the film tube (6) has a wall thickness of less than 0.6 mm, preferably less than 0.3 mm.

6. Catheter (1) according to any of the preceding claims, wherein the material of the film tube (6) comprises a plastics material, preferably a polyurethane.

7. Catheter (1) according to any of the preceding claims, wherein the reinforcement (8) is formed of a guide tube (13) having a tube interior, wherein the guide tube (13) has an outer diameter between 0.5 mm and 2 mm.

8. Catheter (1) according to claim 7, wherein the guide tube (13) is configured to be slid over a guide wire (14) in order to position the catheter (1) as intended.

9. Catheter (1) according to either claim 7 or claim 8, wherein the distal end of the guide tube (13) comprises a medication opening (27).

10. Catheter (1) according to any of the preceding claims, wherein the film tube (6) has a plurality of second openings (5), at least some of which are arranged so as to be spaced apart from the distal end of the film tube (6).

11. Catheter (1) according to claim 10, wherein the film tube (6) has a distal portion (16), which is in particular widened in the shape of a bubble, wherein the second openings (5) are arranged so as to be distributed within this portion.

12. Catheter (1) according to any of the preceding claims,
wherein the pump chamber (18) has a frame (19), the frame having a composition which comprises a shape-memory alloy, in particular nitinol, a shape-memory polymer or a shape-memory ceramic material.

## Revendications

1. Cathéter (1) permettant le guidage ciblé d'un fluide corporel, en particulier du sang, comprenant :
- une section de guidage (2) comportant un volume intérieur (3),
- une section de chambre de pompage (17),
- une première ouverture (4) qui relie le volume intérieur (3) à un extérieur (X),
- une deuxième ouverture (5) qui est disposée de manière distale par rapport à la première ouverture (4) et qui relie le volume intérieur (3) à l'extérieur (X),
- une troisième ouverture (20) ;
la section de chambre de pompage (17) comprenant une chambre de pompage (18), un ballon (21) étant disposé dans la chambre de pompage (18) et une conduite pour un fluide auxiliaire (22) destiné à gonfler le ballon (21) étant raccordée au ballon (21), et la conduite pour le fluide auxiliaire (22) s'étendant vers l'extérieur à travers la troisième ouverture (20) et pouvant être reliée à une pompe, de sorte que, à l'état de fonctionnement du cathéter (1), le fluide corporel est guidé de manière pulsatile dans le volume intérieur (3) entre la première (4) et la deuxième ouverture (5),
la section de guidage (2) comprenant un tube de film (6) comportant un renfort (8) s'étendant à l'intérieur du tube de film (6),
et le tube de film (6) présentant une section pliable (7), une zone de liaison (9) dans laquelle le tube de film (6) est relié au renfort (8), et une section stabilisée (10) comportant une structuration (11).

2. Cathéter (1) selon la revendication 1, dans lequel la section pliable (7) est pliée à l'état prêt à l'emploi du cathéter (1).

3. Cathéter (1) selon la revendication 1 ou 2, dans lequel la section stabilisée (7) comprend un profil sous forme de nervures (12).

4. Cathéter (1) selon l'une des revendications précédentes, le cathéter (1) étant conçu de telle manière que le fluide corporel est aspiré dans le volume intérieur à travers la première ouverture (4), qu'il est guidé dans le volume intérieur (3) dans la direction distale (d) et qu'il est à nouveau évacué du volume intérieur (3) à travers la deuxième ouverture (5).

5. Cathéter (1) selon l'une des revendications précédentes, dans lequel le tube de film (6) présente une épaisseur de paroi inférieure à 0,6 mm, de préférence inférieure à 0,3 mm.

6. Cathéter (1) selon l'une des revendications précédentes, dans lequel le matériau du tube de film (6) comprend une matière plastique, de préférence un polyuréthane.

7. Cathéter (1) selon l'une des revendications précédentes, dans lequel le renfort (8) est formé à partir d'un tube de guidage (13) comportant un intérieur de tube, le tube de guidage (13) présentant un diamètre extérieur compris entre 0,5 et 2 mm.

8. Cathéter (1) selon la revendication 7, dans lequel le tube de guidage (13) est conçu pour être poussé sur un fil de guidage (14) pour le positionnement prévu du cathéter (1).

9. Cathéter (1) selon la revendication 7 ou 8, dans lequel l'extrémité distale du tube de guidage (13) comprend une ouverture pour médicament (27).

10. Cathéter (1) selon l'une des revendications précédentes, dans lequel le tube de film (6) présente une pluralité de deuxièmes ouvertures (5) qui sont disposées au moins partiellement à une distance de l'extrémité distale du tube de film (6).

11. Cathéter (1) selon la revendication 10, dans lequel le tube de film (6) présente une section distale (16), en particulier élargie en forme de bulle, les deuxièmes ouvertures (5) étant réparties dans ladite section.

12. Cathéter (1) selon l'une des revendications précédentes, dans lequel la chambre de pompage (18) présente un cadre (19), une composition du cadre comprenant un alliage à mémoire de forme, en particulier du nitinol, un polymère à mémoire de forme ou une céramique à mémoire de forme.
